# FASCICULE DE BREVET EUROPEEN

(11) **EP 0 518 773 B1**
(45) Date de publication et mention de la délivrance du brevet: **08.02.1995**
(21) Numéro de dépôt: 92401627.2
(22) Date de dépôt: 12.06.1992
(51) Int. Cl.: A61K 7/00, A61K 7/42, A61K 7/13

(54) **Composition cosmétique contenant un mélange de nanopigments d'oxydes métalliques et de pigments mélaniques**
Kosmetisches Mittel enthaltend eine Mischung aus Metalloxide Nanopigmente und Melaninpigmente
Cosmetic composition containing a blend of metallic oxids nanopigments and melanic pigments

(30) Priorité: 14.06.1991 FR 9107324
(43) Date de publication de la demande: 16.12.1992
(73) Titulaire: L'OREAL, 75008 Paris (FR)
(72) Inventeur: Forestier, Serge, F-77410 Claye-Souilly (FR); Hansenne, Isabelle, F-75017 Paris (FR)
(74) Mandataire: Casalonga, Axel

(56) Documents cités:
- EP-A- 0 379 409
- GB-A- 2 207 153
- PATENT ABSTRACTS OF JAPAN, vol. 14, no. 370 (C-747)[4313], 10 août 1990;& JP-A-2 135 275 (TORAY IND. INC.) 24-05-1990

## Description

La présente invention a pour objet une composition cosmétique renfermant des nanopigments d'oxydes métalliques en mélange avec des pigments mélaniques et son utilisation pour la protection de l'épiderme humain et des cheveux et comme produit de maquillage.

L'utilisation d'oxydes métalliques classiques de granulométrie comprise entre 100 et 700 nm tels que l'oxyde de titane dans les produits de maquillage en tant que pigment blanc opacifiant en association avec des pigments colorés est connue. De plus, ces composés sont particulièrement intéressants du fait de leurs propriétés de diffusion et de réflexion du rayonnement ultraviolet, ce qui permet de protéger l'épiderme humain contre les rayons ultraviolets. Cependant, lorsque l'on augmente la concentration en oxyde de titane dans une composition cosmétique afin d'accroître la protection contre les rayons ultraviolets, on obtient un produit cosmétique difficile à étaler sur la peau, opaque et entraînant un blanchiment de la peau.

On a donc essayé de réduire la granulométrie des pigments d'oxydes métalliques. Cependant, on s'est aperçu que l'exposition à la lumière de pigments d'oxydes métalliques de granulométrie inférieure à 100 nm, appelés "nanopigments", peut provoquer une réaction photoinduite préjudiciable à la stabilité des compositions cosmétiques, en particulier celles qui contiennent des lipides.

Selon l'invention, on a découvert d'une façon surprenante que l'addition de pigments mélaniques dans une composition contenant des nanopigments d'oxydes métalliques de granulométrie inférieure à 100 nm permet d'une part, de diminuer ou d'inhiber la réaction photoinduite des nanopigments d'oxydes métalliques et d'autre part, de diminuer le blanchiment de la peau conféré par ces nanopigments.

La présente invention a donc pour objet une composition cosmétique comprenant, en mélange, au moins un nanopigment d'oxydes métalliques et au moins un pigment mélanique, dans un support cosmétiquement acceptable.

Dans la présente demande, on entendra par "nanopigment" un pigment de diamètre moyen inférieur à 100 nm et de préférence compris entre 5 et 50 nm.

Les oxydes métalliques sont choisis parmi les oxydes de titane, de zinc, de cérium, de zirconium ou leurs mélanges.

Les nanopigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Les pigments enrobés sont plus particulièrement des oxydes de titane enrobés :
- de silice tel que le produit "SUNVEIL" de la société IKEDA,
- de silice et d'oxyde de fer tel que le produit "SUNVEIL F" de la société IKEDA,
- de silice et d'alumine tels que les produits "MICROTITANIUM DIOXIDE MT 500 SA" et"MICROTITANIUM DIOXIDE MT 100 SA" de la société TAYCA, "TIOVEIL" de la société TIOXIDE,
- d'alumine tels que les produits "TIPAQUE TTO-55 (B)" et "TIPAQUE TTO-55 (A)" de la société ISHIHARA, et" UVT 14/4" de la société KEMIRA,
- d'alumine et de stéarate d'aluminium tels que le produit "MICROTITANIUM DIOXIDE MT 100 T" de la société TAYCA,
- d'alumine et de laurate d'aluminium tel que le produit "MICROTITANIUM DIOXIDE MT 100 S" de la société TAYCA,
- d'oxyde de fer et de stéarate de fer tel que le produit "MICROTITANIUM DIOXIDE MT 100 F" de la société TAYCA,
- d'oxyde de zinc et de stéarate de zinc tel que le produit "BR 351" de de la société TAYCA,
- de silice, d'alumine et de silicone tels que les produits "MICROTITANIUM DIOXIDE MT 600 SAS " et "MICROTITANIUM DIOXIDE MT 500 SAS" de la société TAYCA,
- de silice, d'alumine, de stéarate d'aluminium et de silicone tel que le produit "STT-30-DS" de la société TITAN KOGYO,
- d'alumine et de silicone tel que le produit "TIPAQUE TTO-55 (S)" de la société ISHIHARA,
- de triéthanolamine tel que le produit "STT-65-S" de la société TITAN KOGYO,
- d'acide stéarique tel que le produit "TIPAQUE TTO-55 (C)" de la société ISHIHARA,
- d'hexamétaphosphate de sodium tel que le produit "MICROTITANIUM DIOXIDE MT 150 W" de la société TAYCA.

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la Société KEMIRA ou enrobé d'alumine, de silice et de glycérine tel que le produit "M 211" vendu par la société KEMIRA.

Les oxydes de titane non enrobés sont par exemple vendus par la société TAYCA sous les dénominations commerciales "MICROTITANIUM DIOXIDE MT 500 B" ou "MICROTITANIUM DIOXIDE MT 600 B", par la société DEGUSSA sous la dénomination "P 25", par la société WACKHERR sous la dénomination "Oxyde de titane transparent PW", par la société MIYOSHI KASEI sous la dénomination "UFTR" et par la société TOMEN sous la dénomination "ITS".

Les oxydes de zinc non enrobés sont par exemple vendus par la société SUMITOMO sous la dénomination "ULTRA FINE ZINC OXIDE POWDER", par la société PRESPERSE sous la dénomination "FINEX 25" ou par la société IKEDA sous la dénomination "MZO-25".

L'oxyde de cérium non enrobé est vendu sous la dénomination "COLLOIDAL CERIUM OXIDE" par la société RHONE POULENC.

Selon l'invention, les nanopigments d'oxyde de titane, enrobés ou non enrobés, sont particulièrement préférés.

Les pigments mélaniques ont un diamètre moyen compris entre 10 nm et 20 000 nm, de préférence entre 30 nm et 15 000 nm.

Le (ou les) pigment(s) mélanique(s) sont dérivés de sources naturelles ou synthétiques et peuvent être obtenus (A) par oxydation d'au moins un composé indolique, (B) par polymérisation oxydante ou enzymatique de précurseurs mélaniques, ou (C) par extraction de la mélanine à partir de substances en contenant.
(A) Les pigments mélaniques peuvent, en premier lieu, être obtenus par oxydation d'au moins un composé indolique choisi notamment parmi ceux répondant à la formule (I): 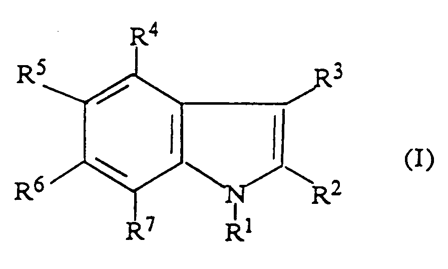 dans laquelle :
   - R¹ et R³ représentent, indépendamment l'un de l'autre, un atome d'hydrogène ou un groupe allyle en C₁-C₄;
   - R² représente un atome d'hydrogène, un groupe allyle en C₁-C₄, un groupe carboxyle ou un groupe alcoxy (C₁-C₄)-carbonyle;
   - R⁴ et R⁷ représentent, indépendamment l'un de l'autre, un atome d'hydrogène, un groupe hydroxy, un groupe allyle en C₁-C₄, un groupe amino, un groupe alcoxy en C₁-C₄, un groupe acyl (C₂-C₄)-oxy ou un groupe acyl (C₂-C₄)-amino;
   - R⁵ représente un atome d'hydrogène, un groupe hydroxy, un groupe alcoxy en C₁-C₄, un groupe alkyle en C₁-C₄, un atome d'halogène, un groupe amino, un groupe acyl (C₂-C₁₄)- oxy, un groupe acyl (C₂-C₄)-amino ou un groupe triméthylsilyloxy;
   - R⁶ représente un atome d'hydrogène, un groupe hydroxy, un groupe alcoxy en C₁-C₄, un groupe amino, un groupe acyl (C₂-C₄)-oxy, un groupe acyl (C₂-C₄)-amino, un groupe triméthylsilyloxy ou un groupe hydroxyalkyl (C₂-C₄)-amino;
   - R⁵ et R⁶ pouvant également former, conjointement avec les atomes de carbone auxquels ils sont rattachés, un cycle méthylènedioxy éventuellement substitué par un groupement alkyle en C₁-C₄ ou alcoxy en C₁-C₄, ou bien un cycle carbonyldioxy;
   - au moins l'un des radicaux R⁴ à R⁷ représente un groupement OZ ou NHR^{0,} l'un au plus des radicaux R⁴ à R⁷ représentant NHR⁰ et deux au plus des radicaux R⁴ à R⁷ représentant OZ et, dans le cas où Z représente un atome d'hydrogène, les deux groupes OH sont dans les positions 5 et 6; et au moins l'un des radicaux R⁴ à R⁷ représente un atome d'hydrogène, et dans le cas où un seul de ces radicaux représente un atome d'hydrogène, un seul radical parmi les radicaux R⁴ à R⁷ représente alors NHR⁰ ou OZ, les autres radicaux représentant un groupe alkyle en C₁-C₄;
   - le radical R⁰ du groupement NHR⁰ désignant un atome d'hydrogène, un groupe acyle en C₂-C₄ ou hydroxyalkyle en C₂-C₄, et le radical Z du groupement OZ désignant un atome d'hydrogène, un groupe acyle en C₂-C₁₄, un groupe alkyle en C₁-C₄, ou un groupe triméthylsilyle,
      et leurs sels de métaux alcalins, alcalino-terreux, d'ammonium ou d'amines, ainsi que les chlorhydrates, bromhydrates, sulfates et méthane sulfonates.

   Les composés indoliques de formule (I) ci-dessus sont choisis, de préférence, parmi le 4-hydroxyindole, le 5-hydroxyindole, le 6-hydroxyindole, le 7-hydroxyindole, le 4-hydroxy-5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthyl indole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6,-diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole, le 5,6-dibenzyloxyindole, et les sels d'addition de ces composés.
   Le 5,6-dihydroxyindole est particulièrement préféré.
   L'oxydation du composé indolique de formule (I) peut s'effectuer en milieu aqueux ou eau-solvant(s), à l'air, en présence ou non d'un agent alcalin et/ou d'un catalyseur métallique d'oxydation tel que par exemple, l'ion cuivrique.
   Le milieu réactionnel est, de préférence, constitué par de l'eau et peut, le cas échéant, être constitué par un mélange d'eau et d'au moins un solvant choisi de telle façon qu'il solubilise rapidement le composé indolique de formule (I). Parmi ces solvants, on peut citer, à titre d'exemples, les alcools inférieurs en C₁-C₄, tels que l'alcool éthylique, l'alcool propylique ou isopropylique, l'alcool tert.-butylique, les alkylèneglycols, tels que l'éthylèneglycol, le propylèneglycol, les alkyléthers d'alkylèneglycols, tels que les éthers monométhylique, monoéthylique et monobutylique de l'éthylèneglycol, les monométhyléthers du propylèneglycol et du dipropylèneglycol, et le lactate de méthyle.
   L'oxydation peut également s'effectuer en mettant en oeuvre le peroxyde d'hydrogène en présence d'un agent alcalin, tel que, de préférence, l'ammoniaque, ou en présence d'un ion iodure, l'iodure étant, de préférence, l'iodure d'un métal alcalin, alcalino-terreux ou d'ammonium.
   On peut également procéder à l'oxydation en utilisant l'acide periodique et ses sels hydrosolubles et dérivés, les permanganates et bichromates, tels que de sodium ou de potassium, l'hypochlorite de sodium, le ferricyanure de potassium, le persulfate d'ammonium, l'oxyde d'argent, l'oxyde de plomb, le chlorure ferrique, le nitrite de sodium, les sels de terres rares dont, notamment le cérium, et des oxydants organiques choisis parmi les ortho- et parabenzoquinones, les ortho- et parabenzoquinones mono- ou diimines, les 1,2- et 1,4-naphtoquinones, les 1,2- et 1,4-naphtoquinones mono- ou diimines telles que définies dans la demande EP-A-0 376 776. Le sel d'acide periodique préféré est le periodate de sodium.
   Il est possible d'activer les agents oxydants par un modificateur de pH.
   On peut également envisager une oxydation enzymatique.
   Le produit insoluble est isolé par filtration, centrifugation, lyophilisation ou atomisation; il est ensuite broyé ou micronisé pour atteindre la granulométrie désirée.
(B) Les pigments mélaniques selon l'invention peuvent également provenir de la polynérisation oxydante ou enzymatique de précurseurs mélaniques, tels que la L-tyrosine, la L-dopa, le catéchol et leurs dérivés.
(C) Les pigments mélaniques selon l'invention peuvent provenir de l'extraction de la mélanine de substances naturelles telles que les cheveux humains, l'encre de céphalopodes (seiches, poulpes), encore connue sous le nom de sépiomélanine, auquel cas le pigment est broyé et purifié avant son utilisation.
(D) Les pigments mélaniques selon l'invention peuvent enfin être obtenus par culture de microorganismes. Ces microorganismes produisent de la mélanine soit naturellement, soit par modification génétique ou par mutagénèse. Des modes de préparation de ces mélanines sont décrits par exemple dans la demande de brevet WO 90-04029.

Le (ou les) pigment(s) mélanique(s) peut ou (peuvent) être présent(s) à la surface ou incorporé(s) dans une charge particulaire inerte, minérale ou organique, pour constituer un pigment mélanique composite synthétique formé in situ. Dans ce cas, le (ou les) pigment(s) mélanique(s) peut (ou peuvent) résulter de l'oxydation d'au moins un composé indolique de formule (I), telle que définie ci-dessus, en mélange avec la charge, dans un milieu essentiellement non-solvant de ladite charge, à une température pouvant aller de la température ambiante jusqu'à environ 100°C, ou encore résulter de la polymérisation oxydante de précurseurs mélaniques sur la charge.

Les conditions générales de l'oxydation des composés indoliques de formule (I) sont les mêmes que celles mentionnées ci-dessus.

Selon un premier mode de réalisation, la charge particulaire est une charge minérale inerte constituée avantageusement de particules de granulométrie inférieure à 20 000 nm. De tels pigments mélaniques composites, déposés sur charge minérale, sont décrits, ainsi que leur préparation, dans la demande de brevet français FR-A-2 618 069.

Selon un deuxième mode de réalisation de la présente invention, la charge particulaire est une charge polymérique inerte, choisie avantageusement parmi les polymères naturels ou synthétiques, organiques ou inorganiques, à réseau réticulé, cristallin ou amorphe, ayant un poids moléculaire compris entre 5000 et 5 000 000. Des pigments mélaniques composites sur charge polymérique ainsi que leur préparation sont décrits dans la demande de brevet européen n° 0379409.

Les polymères organiques ou synthétiques sont, en particulier, choisis parmi les polymères dérivés de la kératine, de la fibroïne de soie, de la chitine ou de la cellulose, ou parmi les polyamides ou les homo- ou copolymères résultant de la polymérisation de monomères mono- ou polyéthyléniques, aliphatiques ou aromatiques, à réseau réticulé, cristallin ou amorphe.

Les polymères dérivés de la kératine sont choisis parmi les kératines animales ou humaines. D'autres polymères dérivés de la kératine utilisables sont des kératines modifiées chimiquement, ayant un poids moléculaire compris entre 10 000 et 250 000 et, en particulier, la kératine partiellement hydrolysée (ou hydrolysat de kératine), ayant un poids moléculaire compris entre 50 000 et 200 000; cet hydrolysat est, de préférence, obtenu par hydrolyse alcaline modérée; des produits de ce type sont, par exemple, vendus sous la dénomination de "KERASOL" par la société CRODA. D'autres kératines modifiées sont les kératines sulfoniques d'un poids moléculaire compris entre 10 000 et 100 000, obtenues par oxydation de tout ou partie des liaisons disulfure des groupements cystine de la kératine en groupements acide cystéique.

Les polymères dérivés de la chitine comprennent d'abord la chitine, qui est un polymère naturel, et le dérivé désacétylé de la chitine connu sous la dénomination de chitosane, obtenu par hydrolyse des groupements acétyle de la chitine. Le chitosane, tel que proposé dans le commerce, est partiellement acétylé et contient 70 à 90% en poids de chitosane. On peut également l'utiliser sous la forme de sels insolubles, tels que les sulfates et phosphates. Des produits de ce typo sont vendus, par exemple, sous la dénomination de "KYTEX" par la société HERCULES.

Les polymères cellulosiques sont choisis plus particulièrement parmi les celluloses microcristallines, telles que les produits vendus sous la dénomination "AVICEL" par la société FMC CORPORATION.

Parmi les polymères synthétiques, on peut tout particulièrement citer le polyéthylène, le polypropylène, le polystyrène, le poly(méthacrylate de méthyle) vendus sous les dénominations "MICROPEARL M" et "MICROPEARL M100" par la société SEPPIC, le poly(méthacrylate de méthyle) réticulé, tel que le produit vendu sous la dénomination de "MICROPEARL M 305" par la société SEPPIC. D'autres polymères sont, en particulier, choisis parmi la poly-β-alanine réticulée, telle que décrite dans le brevet français 2 530 250 dont le taux de réticulation est compris entre 1 et 15% et, de préférence, entre 1 et 8%.

On peut également utiliser, à titre de polymères, des produits connus sous la dénomination de microéponges, tels que des polymères réticulés de styrène/divinylbenzène ou de méthacrylate de méthyle/diméthacrylate d'éthylène-glycol ou de stéarate de vinyle/divinylbenzène, tels que décrits dans les brevets WO-88/01164 et US-A-4 690 825. De tels polymères sont essentiellement constitués par des billes de polymères réticulés comprenant un réseau interne de pores, capable de retenir le pigment mélanique. D'autres polymères de ce type sont des microsphères creuses d'un copolymère de chlorure de vinylidène et d'acrylonitrile, vendues sous la dénomination d'"EXPANCEL" par la Société KEMA NORD; ou encore des microsphères poreuses de polyamide 12, de polyamide 6 ou de copolyamide 6/12, vendues sous la dénomination d'"ORGASOL" par la société ATOCHEM.

On peut utiliser également des poudres de silicone qui sont des gommes, des résines et, plus particulièrement, des élastomères d'organopolysiloxane.

Les nanopigments d'oxydes métalliques sont avantageusement présents dans la composition cosmétique selon l'invention à une concentration comprise entre 0,1 et 15% en poids par rapport au poids total de la composition, et de préférence comprise entre 0,5 et 10%.

Les pigments mélaniques sont avantageusement présents dans la composition cosmétique selon l'invention à une concentration comprise entre 0,001 et 2%, de préférence entre 0,002 et 1% en poids par rapport au poids total de la composition.

Le rapport en poids pigment mélanique/nanopigment d'oxyde métallique est avantageusement compris entre 0,00007 et 10, de préférence entre 0,001 et 0,1.

La composition cosmétique de l'invention peut être utilisée comme composition protectrice de l'épiderme humain ou des cheveux contre les rayons ultraviolets, comme composition antisolaire ou comme produit de maquillage.

Cette composition peut se présenter en particulier sous forme de lotion, de lotion épaissie, de gel, de crème, de lait, de poudre, de bâtonnet solide et éventuellement être conditionnée en aérosol et se présenter sous forme de mousse ou de spray.

Elle peut contenir les adjuvants cosmétiques habituellement utilisés tels que des corps gras, des solvants organiques, des silicones, des épaississants, des adoucissants, des filtres solaires UV-A, UV-B, ou à bande large, des agents anti-mousses, des agents hydratants, des parfums, des conservateurs, des tensio-actifs, des charges, des séquestrants, des polymères anioniques, cationiques, non-ioniques, amphotères, ou leurs mélanges, des propulseurs, des agents alcalinisants ou acidifiants, des colorants, des pigments de granulométrie supérieure à 100 nm comme les oxydes de fer, ou tout autre ingrédient habituellement utilisé en cosmétique.

Parmi les solvants organiques, on peut citer les alcools et polyols inférieurs tels que l'éthanol, l'isopropanol, le propylène glycol, la glycérine et le sorbitol.

Les corps gras peuvent être constitués par une huile ou une cire ou leur mélange, les acides gras, les esters d'acides gras, les alcools gras, la vaseline, la paraffine, la lanoline, la lanoline hydrogénée, la lanoline acétylée.

Les huiles sont choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin, les huiles de silicone et les isoparaffines.

Les cires sont choisies parmi les cires animales, fossiles, végétales, minérales ou de synthèse. On peut citer notamment les cires d'abeille, les cires de Carnauba, de Candelila, de canne à sucre, du Japon, les ozokérites, la cire de Montan, les cires microcristallines, les paraffines, les cires et résines de silicone.

Les esters d'acides gras sont par exemple le myristate d'isopropyle, l'adipate d'isopropyle, le palmitate d'isopropyle, le palmitate d'octyle, les benzoates d'alcools gras en C₁₂-C₁₅ ("FINSOLV TN" de FINETEX), l'alcool myristique oxypropyléné à 3 moles d'oxyde de propylène ("WITCONOL APM" de WITCO), les triglycérides d'acides caprique et caprylique ("MIGLYOL 812" de HULS).

La composition cosmétique selon l'invention peut contenir des épaississants qui peuvent être choisis parmi les polymères d'acide acrylique réticulés ou non, et particulièrement les acides polyacryliques réticulés par un agent polyfonctionnel tels que les produits vendus sous la dénomination "CARBOPOL" par la société GOODRICH, les dérivés de cellulose tels que la méthylcellulose, l'hydroxyéthylcellulose, l'hydroxypropylinéthylcellulose, les sels de sodium de la carboxyméthylcellulose, les mélanges d'alcool cétylstéarylique et d'alcool cétylstéarylique oxyéthyléné à 33 moles d'oxyde d'éthylène.

On peut également utiliser les produits résultant de l'interaction ionique d'un polymère cationique constitué par un copolymère de cellulose ou d'un dérivé de cellulose greffés par un sel de monomère hydrosoluble d'ammonium quaternaire et d'un polymère anionique carboxylique tels que décrits dans le brevet français FR-2 598 611. On utilise de préférence le produit d'interaction ionique d'un copolymère d'hydroxyéthylcellulose greffé par voie radicalaire par du chlorure de diallyldiméthlylammoniun tel que le polymère commercialisé sous la dénomination "CELQUAT L 200" par la société National Starch avec, soit des copolymères d'éthylène et d'anhydride maléique tels que les produits vendus sous la dénomination "EMA 31" par la société MONSANTO, soit des copolymères 50/50 d'acide méthacrylique et de méthacrylate de méthyle.

Un autre produit de ce type utilisable est le produit résultant de l'interaction ionique du copolymère d'hydroxyéthylcellulose greffé par voie radicalaire par du chlorure de diallyldiméthylammonium avec un polymère anionique carboxylique réticulé tel que les copolymères de l'acide méthacrylique et de l'acrylate d'éthyle réticulés vendus sous la dénomination "VISCOATEX" 538, 46 ou 50 par la société COATEX.

Lorsque la composition cosmétique selon l'invention est utilisée pour la protection de l'épiderme humain contre les rayons UV ou comme composition antisolaire, elle peut se présenter sous forme de suspension ou de dispersion dans des solvants ou des corps gras, ou encore sous forme d'émulsion telle qu'une crème ou un lait, sous forme de pommade, de gel, de bâtonnet solide ou de mousse aérosol. Les émulsions peuvent contenir en outre des agents tensio-actifs anioniques, non-ioniques, cationiques ou amphotères.

Lorsque la composition cosmétique selon l'invention est utilisée pour la protection des cheveux, elle peut se présenter sous forme de shampooing, de lotion, de gel ou composition à rincer, à appliquer avant ou après shampooing, avant ou après coloration ou décoloration, avant, pendant ou après permanente ou défrisage, de lotion ou gel coiffants ou traitants, de lotion ou gel pour le brushing ou la mise en plis, de laque pour cheveux, de composition de permanente ou de défrisage, de coloration ou décoloration des cheveux.

Lorsque la composition est utilisée conne produit de maquillage des cils, des sourcils, de la peau ou des cheveux, tel que crème de traitement de l'épiderme, fond de teint, bâton de rouge à lèvres, fard à paupières, fard à joues, ligneur encore appelé "eye-liner", mascara, gel colorant, elle peut se présenter sous forme solide ou pâteuse, anhydre ou aqueuse, comme des émulsions huile-dans-eau ou eau-dans-huile, des suspensions ou encore des gels.

L'invention a aussi pour objet un procédé de protection de l'épiderme humain et des cheveux contre le rayonnement ultraviolet ainsi qu'un procédé de maquillage consistant à appliquer sur la peau ou les cheveux une quantité efficace de la composition cosmétique ci-dessus.

L'invention a également pour objet l'utilisation des pigments mélaniques dérivés de sources naturelles ou synthétiques, de diamètre moyen compris entre 10 nm et 20 000 nm, pour diminuer ou inhiber la réaction photoinduite des nanopigments d'oxydes métalliques exposés à la lumière, ces oxydes métalliques étant choisis parmi les oxydes de titane, de zinc, de cérium, de zirconium ou leurs mélanges ayant un diamètre moyen inférieur à 100nm et de préférence compris entre 5 et 50 nm.

L'invention sera mieux illustrée par les exemples non limitatifs ci-après.

### EXEMPLE 1

On prépare une émulsion antisolaire huile-dans-eau de composition suivante:

| | |
|---|---|
| - Pigment mélanique obtenu par oxydation du 5,6-dihydroxyindole | 0,1 g |
| - Oxyde de titane enrobé d'alumine et de stéarate d'aluminium vendu sous la dénomination "MICROTITANIUM DIOXIDE MT 100T" par la société TAYCA | 6,5 g |
| - Mélange d'alcool cétylstéarylique et d'alcool cétylstéarylique oxyéthyléné à 33 mole d'oxyde d'éthylène vendu sous la dénomination "SINNOWAX AO" par la société HENKEL | 7,0 g |
| - Mélange de mono- et distéarate de glycérol non autoémulsionable | 2,0 g |
| - Huile de vaseline | 15,0 g |
| - Alcool cétylique | 1,5 g |
| - Polydiméthylsiloxane | 1,5 g |
| - Glycérine | 20,0 g |
| - Conservateurs, parfum qs | |
| - Eau qsp | 100 g |

### EXEMPLE 2

On prépare une émulsion antisolaire huile-dans-eau de composition suivante :

| | |
|---|---|
| - Pigment mélanique obtenu par oxydation du 5,6-dihydroxyindole | 0,015 g |
| - Oxyde de titane enrobé d'alumine et de stéarate d'aluminium vendu sous la dénomination "MICROTITANIUM DIOXIDE MT 100T" par la société TAYCA | 6,5 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mélange de mono- et distéarate de glycérol non autoémulsionnable | 2,0 g |
| - Huile de vaseline | 15,0 g |
| - Alcool cétylique | 1,5 g |
| - Polydiméthylsiloxane | 1,5 g |
| - Glycérine | 20,0 g |
| - Conservateurs, parfum qs | |
| - Eau qsp | 100 g |

### EXEMPLE 3

On prépare une émulsion antisolaire huile-dans-eau de composition suivante:

| | |
|---|---|
| - Pigment mélanique obtenu par oxydation du 5,6-dihydroxyindole | 0,1 g |
| - Oxyde de titane enrobé d'alumine et de stéarate d'aluminium vendu sous la dénomination "MICROTITANIUM DIOXIDE MT 100T" par la société TAYCA | 6,5 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Mélange de mono- et distéarate de glycérol non autoémulsionnable | 2,0 g |
| -Huile de vaseline | 15,0 g |
| - Alcool cétylique | 1,5 g |
| - Polydiméthylsiloxane | 1,5 g |
| - Glycérine | 20,0 g |
| - Oxyde de fer jaune | 0,4 g |
| - Oxyde de fer rouge | 0,2g |
| - Conservateurs, parfum qs | |
| - Eau qsp | 100 g |

### EXEMPLE 4

On prépare une émulsion antisolaire eau-dans-l'huile de composition suivante :

| | |
|---|---|
| - Pigment mélanique obtenu par oxydation du 5,6-dihydroxyindole | 0,3 g |
| - Oxyde de zinc vendu sous la dénomination "ULTRA FINE ZINC OXIDE POWDER" par la société SUMITOMO | 3,0 g |
| - Huile de vaseline | 15,0 g |
| - Hydroxystéarate de sorbitan et de glycérol oxyéthyléné à 2,5 mole d'oxyde d'éthylène et oxypropyléné à 1,5 moles d'oxyde de propylène vendu sous la dénomination "ARLACEL 780" par la société ICI | 5,0 g |
| - Sulfate de magnésium | 0,7 g |
| - Conservateur qs | |
| - Eau qsp | 100 g |

### EXEMPLE 5

On prépare une émulsion antisolaire eau-dans-l'huile de composition suivante :

| | |
|---|---|
| - Pigment mélanique obtenu par oxydation du 5,6-dihydroxyindole | 0,002 g |
| - Oxyde de titane enrobé d'alumine et de stéarate d'aluminium vendu sous la dénomination "MICROTITANIUM DIOXIDE MT 100T" par la société TAYCA | 6,5 g |
| - Huile de vaseline | 15,0 g |
| - Hydroxystéarate de sorbitan et de glycérol oxyéthyléné à 2,5 moles d'oxyde d'éthylène et oxypropyléné à 1,5 moles d'oxyde de propylène vendu sous la dénomination "ARLACEL 780" par la société ICI | 5,0 g |
| - Sulfate de magnésium | 0,7 g |
| - Conservateur qs | |
| - Eau qsp | 100 g |

### EXEMPLE 6

On prépare une crème teintée de composition suivante :

| | |
|---|---|
| - Mélange de mon- et distéarate de glycérol non autoémulsionnable | 3,5 g |
| - Isostéarate de glycérol | 1,8 g |
| - Mélange d'huile minérale et d'alcool de lanoline vendu sous la dénomination "AMERCHOL L-101" par la société AMERCHOL | 3,1 g |
| - Palmitate d'isopropyle | 7,6 g |
| - Palmitate d'octyle | 7,0 g |
| - Ultramarine violet | 0,75 g |
| - Dioxyde de titane de granulométrie 200-300 nm | 3,0 g |
| - Oxyde de fer jaune | 1,0 g |
| - Oxyde de fer rouge | 0,6 g |
| - Oxyde de fer noir | 0,08 g |
| - Conservateurs | 0,5 g |
| - Parfum | 0,3 g |
| - Silicate d'aluminium et de magnésium | 1,5 g |
| - Talc | 4,46 g |
| - Triéthanolamine | 1,2 g |
| - Gomme de cellulose | 0,05 g |
| - Gomme de xanthane | 0,15 g |
| - Cyclométhicone (dénomination "CTFA Dictionary"; diméthyl polysiloxane cyclique) | 7,5 g |
| - Propylène glycol | 3,0 g |
| - Glycérine | 2,0 g |
| - Acide stéarique | 2,5 g |
| - Dioxyde de titane de granulométrie 30-40 nm | 6,0 g |
| - Pigment mélanique obtenu par oxydation du 5,6-dihydroxyindole | 0,02 g |
| - Eau qsp | 100 g |

On chauffe séparément à 80°C la phase grasse contenant les huiles et l'acide stéarique et la phase aqueuse contenant la triéthanolamine.

Le mélange est émulsionné à 80°C et refroidi lentement. Pendant le refroidissement, sont ajoutés : le mélange de pigments préalablement broyé dans le propylène glycol et le cyclométhicone.

### EXEMPLE 7

On prépare un fond de teint de composition suivante :

| | |
|---|---|
| - Triéthanolamine | 1,0 g |
| - Stéarate de polyéthylène glycol à 2 moles d'oxyde | 0,53 g |
| d'éthylène | |
| - Mélange de mono- et distéarate de glycérol non autoémulsionnable | 0,35 g |
| - Silicate d'aluminium et de magnésium | 1,5 g |
| - Oxyde de fer jaune | 0,9 g |
| - Oxyde de fer rouge | 0,5 g |
| - Oxyde de fer noir | 0,2 g |
| - Dioxyde de titane de granulométrie 200-300 nm | 5,4 g |
| - Dioxyde de titane de granulométrie 30-40 nm | 8,0 g |
| - Pigment mélanique obtenu par oxydation du 5,6-dihydroxyindole | 0,02 g |
| - Conservateurs | 0,5 g |
| - Mélange de polyéthylène glycol à 6 moles d'oxyde d'éthylène et de polyéthylène glycol à 32 moles d'oxyde d'éthylène vendu sous la dénomination "CARBOWAX 1450" par la société UNION CARBIDE | 9,0 g |
| - Gomme de cellulose | 0,02 g |
| - Polyéthylène | 9,3 g |
| - Cyclométhicone (dénomination "CTFA Dictionary"; diméthylpolysiloxane cyclique) | 14,0 g |
| - Propylèneglycol | 6,0 g |
| - Glycérine | 3,0 g |
| - Acide stéarique | 2,2 g |
| - Eau qsp | 100 g |

La composition est préparée de façon analogue à l'exemple 6.

### EXEMPLE 8

### Mascara noir

| | |
|---|---|
| - Stéarate de triéthanolamine | 15,0 g |
| - Cire d'abeilles | 5,0 g |
| - Paraffine | 3,0 g |
| - Cire de Carnauba | 10,0 g |
| - Parahydroxybenzoate de propyle | 0,2 g |
| - Parahydroxybenzoate de méthyle | 0,2 g |
| - Gomme arabique | 3,0 g |
| - Hydroxyéthylcellulose | 0,3 g |
| - Pigment mélanique obtenu par oxidation de 5,6-dihydroxyindole | 1,0 g |
| - Dioxyde de titane de granulométrie moyenne 30-40 nm | 2,0 g |
| - Eau qsp | 100 g |

### EXEMPLE 9

On prépare une émulsion antisolaire huile-dans-eau de composition suivante :

| | |
|---|---|
| - Oxyde de cérium (diamètre moyen 12 nanomètres) en suspension aqueuse à 20% MA vendu sous la dénomination "COLLOIDAL CERIUM OXIDE" par la société RHONE POULENC | 3,0 g MA |
| - Pigment mélanique obtenu par oxydation du 2-méthyl 5,6-dihydroxyindole | 0,05 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Stéarate de glycérol vendu sous la dénomination "GELEOL" par la société GATTEFOSSE | 2,0 g |
| - Alcool cétylique | 1,5 g |
| - Huile de vaseline | 15,0 g |
| - Glycérine qs | 3,0 g |
| - Eau qsp | 100 g |

### EXEMPLE 10

On prépare un gel de coiffage protecteur du rayonnement UV et colorant de composition suivante :

| | |
|---|---|
| - Dioxyde de titane (diamètre : 15 à 40 nanomètres) vendu sous la dénomination "P 25" par la société DEGUSSA | 0,2 g |
| - Copolymère d'acide méthacrylique et d'acrylate d'éthyle réticulé vendu à 30% de MA sous la dénomination "VISCOATEX 46" par la société COATEX | 1,35 g MA |
| - Copolymère d'hydroxyéthylcellulose et de chlorure de diallyl diméthyl ammonium vendu sous la dénomination "CELQUAT L 200" par la société NATIONAL STARCH | 1,0 g |
| - Pigment mélanique obtenu par oxydation du 5,6-dihydroxyindole | 1,0 g |
| - Polymère cationique siliconé vendu à la concentration de 35% de MA sous la dénomination "Emulsion cationique DC929" par la société DOW CORNING | 0,3 g |
| - Amino-2 méthyl-2 propanol-1 qs pH 7,5 | |
| - Parfum, conservateur qs | |
| - Eau qsp | 100 g |

### EXEMPLE 11

On prépare une émulsion antisolaire huile-dans-eau de composition suivante :

| | |
|---|---|
| - Oxyde de titane enrobé d'alumine et de stéarate d'aluminium vendu sous la dénomination "MICROTITANIUM DIOXIDE MT 100T" par la société TAYCA | 5,0 g |
| - Pigment mélanique obtenu par oxydation du bromhydrate de 2-méthyl 5,6-dihydroxyindole | 0,1 g |
| - Mélange d'alcool cétylstéarylique et d'alcool cétylstéarylique oxyéthyléné à 33 mole d'oxyde d'éthylène vendu sous la dénomination "SINNOWAX AO" par la société HENKEL | 7,0 g |
| - Stéarate de glycérol vendu sous la dénomination "GELEOL" par la société GATTEFOSSE | 2,0 g |
| - Alcool cétylique | 1,5 g |
| - Huile de vaseline | 15,0 g |
| - Glycérine | 3,0 g |
| - Conservateur, parfum qs | |
| - Eau qsp | 100 g |

### EXEMPLE 12

On prépare une émulsion antisolaire huile-dans-eau de composition suivante :

| | |
|---|---|
| - Oxyde de titane enrobé d'alumine et de stéarate d'aluminium vendu sous la dénomination "MICROTITANIUM DIOXIDE MT 100T" par la société TAYCA | 5,0 g |
| - Pigment mélanique obtenu par oxydation du 2-méthyl 5,6-dihydroxyindole | 0,1 g |
| - Mélange d'alcool cétylstéarylique et d'alcool cétylstéarylique oxyéthyléné à 33 moles d'oxyde d'éthylène vendu sous la dénomination "SINNOWAX AO" par la société HENKEL | 7,0 g |
| - Stérate de glycérol vendu sous la dénomination "GELEOL" par la société GATTEFOSSE | 2,0 g |
| - Alcool cétylique | 1,5 g |
| - Huile de vaseline | 15,0 g |
| - Glycérine | 3,0 g |
| - Conservateur, parfum qs | |
| - Eau qsp | 100 g |

### EXEMPLE 13

### Gel de coiffage

| | |
|---|---|
| - Copolymère de polyvinylpyrrolidone et d'acétate de vinyle (65/35 en poids) vendu par la société GAF sous la dénomination PVP/VA S 630 | 0,5 g |
| - Acide polyacrylique réticulé vendu sous la dénomination "CARBOPOL 940" (PM 4 000 000) par la société GOODRICH | 0,5 g |
| - Dioxyde de titane vendu par la société DEGUSSA sous la dénomination "P 25" | 0,2 g |
| - Pigment mélanique obtenu par oxydation du 5,6-dihydroxyindole | 2,0 g |
| - Alcool éthylique | 17,2 g |
| - Parfum, colorant, conservateur qs | |
| - Triéthanolamine qs pH : 7,5 | |
| - Eau qsp | 100 g |

## Revendications

1. Composition cosmétique caractérisée par le fait qu'elle comprend, en mélange, au moins un nanopigment d'oxydes métalliques choisis parmi les oxydes de titane, de zinc, de cérium, de zirconium ou leurs mélanges, de diamètre moyen inférieur à 100 nm et au moins un pigment mélanique dérivé de sources naturelles ou synthétiques de diamètre moyen compris entre 10 nm et 20 000 nm, dans un support cosmétiquement acceptable.

2. Composition cosmétique selon la revendication 1, caractérisée par le fait que les nanopigments d'oxydes métalliques ont un diamètre moyen compris entre 5 et 50 nm.

3. Composition cosmétique selon la revendication 1 ou 2, caractérisée par le fait que l'oxyde métallique est l'oxyde de titane.

4. Composition cosmétique selon l'une quelconque des revendications 1 ou 3, caractérisée par le fait que les pigments mélaniques ont un diamètre moyen compris entre 30 nm et 15 000 nm.

5. Composition cosmétique selon l'une quelconque des revendications 1 à 4, caractérisée par le fait que les nanopigments d'oxydes métalliques sont présents dans la composition à une concentration comprise entre 0,1 et 15% en poids par rapport au poids total de la composition, et de préférence comprise entre 0, 5 et 10% en poids.

6. Composition cosmétique selon l'une quelconque des revendications 1 à 5, caractérisée par le fait que les pigments mélaniques sont présents dans la composition à une concentration comprise entre 0,001 et 2% en poids, et de préférence entre 0,002 et 1% en poids, par rapport au poids total de la composition.

7. Composition cosmétique selon l'une quelconque des revendications 1 à 6, caractérisée par le fait que le rapport en poids pigment mélanique/nanopigment d'oxyde métallique est compris entre 0,00007 et 10, et de préférence entre 0,001 et 0,1.

8. Composition cosmétique selon l'une quelconque des revendications 1 à 7, caractérisée par le fait que les nanopigments d'oxydes métalliques sont des pigments non enrobés.

9. Composition cosmétique selon l'une quelconque des revendications 1 à 7, caractérisée par le fait que les nanopigments d'oxydes métalliques sont des pigments enrobés ayant subi un ou plusieurs traitements de surface de nature chimique, électronique, mécanochimique ou mécanique avec des composés choisis parmi les aminoacides, la cire d'abeille, les acides gras, les alcools gras, les tensio-actifs anioniques, les lécithines, les sels de sodium, potassium, zinc, fer ou aluminium d'acides gras, l'hexamétaphosphate de sodium, les alcoxydes métalliques, le polyéthylène, les silicones, les protéines, les alcanolamines, les oxydes de silicium et les oxydes métalliques.

10. Composition cosmétique selon la revendication 9, caractérisée par le fait que les nanopigments d'oxydes métalliques enrobés sont des pigments d'oxydes de titane enrobés de silice, de silice et d'alumine, de silice et d'oxyde de fer, d'alumine et de silicone, d'alumine, d'alumine et de stéarate d'aluminium, d'alumine et de laurate d'aluminium, d'oxyde de fer et de stéarate de fer, d'oxyde de zinc et de stéarate de zinc, de silice et d'alumine et de silicone, de silice et d'alumine et de stéarate d'aluminium et de silicone, de triéthanolamine, d'acide stéarique ou d'hexamétaphosphate de sodium..

11. Composition cosmétique selon la revendication 9, caractérisée par le fait que les nanopigments d'oxydes métalliques enrobés sont des mélanges de dioxyde de titane et de dioxyde de cérium enrobés de silice ou des mélanges de dioxyde de titane et de dioxyde de zinc enrobés d'alumine, de silice et de silicone ou enrobés d'alumine, de silice et de glycérine.

12. Composition cosmétique selon l'une quelconque des revendications 1 à 11, caractérisée par le fait que les pigments mélaniques sont obtenus par oxydation d'au moins un composé indolique, par polymérisation oxydante ou enzymatique de précurseurs mélaniques ou par extraction de la mélanine à partir de substances en contenant.

13. Composition cosmétique selon la revendication 12, caractérisée par le fait que les pigments mélaniques sont obtenus par oxydation d'au moins un composé indolique de formule 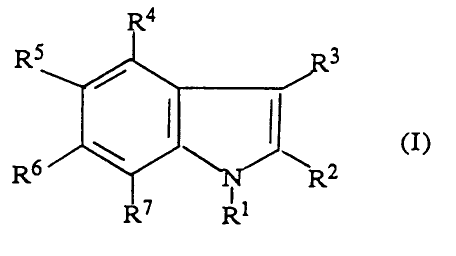 dans laquelle :
- R¹ et R³ représentent, indépendamment l'un de l'autre, un atome d'hydrogène ou un groupe alkyle en C₁-C₄, R² représente un atome d'hydrogène, un groupe alkyle en C₁-C₄, un groupe carboxyle ou un groupe alcoxy (C₁-C₄)-carbonyle; R⁴ et R⁷ représentent, indépendamment l'un de l'autre, un atome d'hydrogène, un groupe hydroxy, un groupe alkyle en C₁-C₄, un groupe amino, un groupe alcoxy en C₁-C₄, un groupe acyl (C₂-C₄)-oxy, ou un groupe acyl (C₂-C₄)-amino; R⁵ représente un atome d'hydrogène, un groupe hydroxy, un groupe alcoxy en C₁-C₄, un groupe alkyle en C₁-C₄, un atome d'halogène, un groupe amino, un groupe acyl (C₂-C₁₄)- oxy, un groupe acyl (C₂-C₄)-amino ou un groupe triméthylsilyloxy; R⁶ représente un atome d'hydrogène, un groupe hydroxy, un groupe alcoxy en C₁-C₄, un groupe amino, un groupe acyl (C₂-C₄)-oxy, un groupe acyl (C₂-C₄)-amino, un groupe triméthylsilyloxy ou un groupe hydroxyalkyl (C₂-C₄)-amino; R⁵ et R⁶ pouvant également former, conjointement avec les atomes de carbone auxquels ils sont rattachés, un cycle méthylènedioxy éventuellement substitué par un groupement alkyle en C₁-C₄ ou alcoxy en C₁-C₄, ou bien un cycle carbonyldioxy; au moins l'un des radicaux R⁴ à R⁷ représente un groupement OZ ou NHR⁰, l'un au plus des radicaux R⁴ à R⁷ représentant NHR⁰ et deux au plus des radicaux R⁴ à R⁷ représentant OZ et, dans le cas où Z représente un atome d'hydrogène, les deux groupes OH sont dans les positions 5 et 6; et au moins l'un des radicaux R⁴ à R⁷ représente un atome d'hydrogène, et dans le cas où un seul de ces radicaux représente un atome d'hydrogène, un seul radical parmi les radicaux R⁴ à R⁷ représente alors NHR⁰ ou OZ, les autres radicaux représentant des groupes alkyle en C₁-C₄; le radical R⁰ du groupement NHR⁰ désignant un atome d'hydrogène, un groupement acyle en C₂-C₄ ou hydroxyalkyle en C₂-C₄, et le radical Z du groupement OZ désignant un atome d'hydrogène, un groupe acyle en C₂-C₁₄, alkyle en C₁-C₄, ou triméthylsilyle;
et les sels de métaux alcalins, alcalino-terreux, d'ammonium ou d'amines ainsi que les chlorhydrates, bromhydrates, sulfates et méthane sulfonates de ce composé indolique.

14. Composition cosmétique selon la revendication 13, caractérisée par le fait que le composé indolique est choisi parmi le 4-hydroxyindole, le 5-hydroxyindole, le 6-hydroxyindole, le 7-hydroxyindole, le 4-hydroxy-5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxy-indole, le 5,6-dihydroxyindole, le N-méthyl 5,6-dihydroxyindole, le 2-méthyl 5,6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthyl indole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N-β-hydroxyéthylaminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino 2,3,4-triméthylindole, le 6-amino 2,3,5-triméthylindole, le 6-amino 2,3,6-triméthylindole, le 5,6,-diacétoxyindole, le 5-méthoxy 6-acétoxyindole, le 5,6-diméthoxyindole, le 5,6-méthylènedioxyindole, le 5,6-triméthylsilyloxyindole, l'ester phosphorique du 5,6-dihydroxyindole, le 5,6-dibenzyloxyindole, et les sels d'addition de ces composés.

15. Composition cosmétique selon la revendication 14, caractérisée par le fait que le composé indolique est le 5,6-dihydroxyindole.

16. Composition cosmétique selon l'une quelconque des revendications 1 à 15, caractérisée par le fait que le pigment mélanique est un pigment mélanique composite synthétique déposé à la surface ou incorporé dans une charge particulaire inerte minérale ou organique.

17. Composition cosmétique selon la revendication 16, caractérisée par le fait que le pigment mélanique composite résulte de l'oxydation d'au moins un composé indolique de formule (I) selon la revendication 13, en mélange avec la charge particulaire, dans un milieu essentiellement non-solvant de ladite charge, à une température allant de la température ambiante jusqu'à environ 100°C, ou encore résulte de la polymérisation oxydante de précurseurs mélaniques sur la charge particulaire.

18. Composition cosmétique selon la revendication 16 ou 17, caractérisée par le fait que la charge particulaire est constituée par des particules minérales inertes de granulométrie inférieure à 20 000 nm ou de polymères naturels ou synthétiques, organiques ou inorganiques à réseau réticulé, cristallin ou amorphe, de poids moléculaire compris entre 5000 et 5 000 000.

19. Composition cosmétique selon l'une quelconque des revendications 1 à 18, caractérisée par le fait qu'elle se présente sous forme de lotion, lotion épaissie, gel, crème, lait, poudre, bâtonnet solide, mousse ou spray.

20. Composition cosmétique selon la revendication 19, caractérisée par le fait qu'elle contient en outre des adjuvants cosmétiques choisis parmi les corps gras, les solvants organiques, les silicones, les épaississants, les adoucissants, les filtres solaires UV-A, UV-B ou à bande large, les agents antimousses, les agents hydratants, les parfums, les conservateurs, les tensio-actifs, les charges, les séquestrants, les polymères anioniques, cationiques, non-ioniques, amphotères ou leurs mélanges, les propulseurs, les agents alcalinisants ou acidifiants, les colorants et les pigments de granulométrie supérieure à 100 nm.

21. Composition cosmétique selon l'une quelconque des revendications 1 à 20, constituant une composition protectrice de l'épiderme humain contre les rayons ultraviolets, ou antisolaire, caractérisée par le fait qu'elle se présente sous forme de suspension ou de dispersion dans des solvants ou des corps gras, sous forme d'émulsion, sous forme de pommade, sous forme de gel, sous forme de bâtonnet solide ou de mousse aérosol.

22. Composition cosmétique selon l'une quelconque des revendications 1 à 20 utilisée pour la protection des cheveux contre les rayons ultraviolets, caractérisée par le fait qu'elle se présente sous forme de shampooing, de lotion, de gel ou composition à rincer, à appliquer avant, ou après shampooing, avant ou après coloration ou décoloration, avant, pendant ou après permanente ou défrisage, sous forme de lotion ou de gel coiffants ou traitants, de lotion ou gel pour le brushing ou la mise en plis, de laque pour cheveux, de composition de permanente ou de défrisage, de coloration ou décoloration des cheveux.

23. Composition cosmétique selon l'une quelconque des revendications 1 à 20 constituant un produit de maquillage des cils, de sourcils, de la peau ou des cheveux, caractérisée par le fait qu'elle se présente sous forme de crème de traitement de l'épiderme, fond de teint, bâton de rouge à lèvres, fard à paupières, fard à joues, ligneur encore appelé "eye-liner", mascara ou gel colorant et se présente sous forme solide ou pâteuse, anhydre ou aqueuse.

24. Procédé de protection non-thérapeutique de l'épiderme humain et des cheveux contre le rayonnement ultraviolet, caractérisé par le fait qu'il consiste à appliquer sur la peau ou les cheveux une quantité efficace d'une composition cosmétique selon l'une quelconque des revendications 1 à 23.

25. Utilisation non-thérapeutique de pigments mélaniques dérivés de sources naturelles ou synthétiques de diamètre moyen compris entre 10 nm et 20 000 nm pour diminuer ou inhiber la réaction photoinduite des nanopigments d'oxydes métalliques exposés à la lumière, ces oxydes métalliques étant choisis parmi les oxydes du titane, de zinc, de cérium, de zirconium ou leurs mélanges, de diamètre moyen inférieur à 100 nm.

## Claims

1. A cosmetic composition which comprises, in a mixture, at least one metal oxide nanopigment chosen from titanium, zinc, cerium or zirconium oxide or mixtures thereof, with a mean diameter of less than 100 nm, and at least one melanin pigment derived from natural or synthetic sources, with a mean diameter of between 10 nm and 20,000 nm, in a cosmetically acceptable carrier.

2. The cosmetic composition as claimed in claim 1, wherein the metal oxide nanopigments have a mean diameter of between 5 and 50 nm.

3. The cosmetic composition as claimed in claim 1 or 2, wherein the metal oxide is titanium oxide.

4. The cosmetic composition as claimed in any one of claims 1 or 3, wherein the melanin pigments have a mean diameter of between 30 and 15,000 nm.

5. The cosmetic composition as claimed in any one of claims 1 to 4, wherein the metal oxide nanopigments are present in the composition at a concentration of between 0.1 and 15 % by weight relative to the total weight of the composition, and preferably of between 0.5 and 10 % by weight.

6. The cosmetic composition as claimed in any one of claims 1 to 5, wherein the melanin pigments are present in the composition at a concentration of between 0.001 and 2 % by weight, and preferably between 0.002 and 1 % by weight relative to the total weight of the composition.

7. The cosmetic composition as claimed in any one of claims 1 to 6, wherein the melanin pigment/metal oxide nanopigment weight ratio is between 0.00007 and 10, preferably between 0.001 and 0.1.

8. The cosmetic composition as claimed in any one of claims 1 to 7, wherein the metal oxide nanopigments are uncoated pigments.

9. The cosmetic composition as claimed in any one of claims 1 to 7, wherein the metal oxide nanopigments are coated pigments having undergone one or more surface treatments of a chemical, electronic, mechanicochemical or mechanical nature, with compounds chosen from amino acids, beeswax, fatty acids, fatty alcohols, anionic surface-active agents, lecithins, sodium, potassium, zinc, iron or aluminum salts of fatty acids, sodium hexametaphosphate, metal alkoxides, polyethylene, silicones, proteins, alkanolamines, silicon oxides and metal oxides.

10. The cosmetic composition as claimed in claim 9, wherein the coated metal oxide nanopigments are titanium oxide pigments coated with silica, silica and alumina, silica and iron oxide, alumina and silicone, alumina, alumina and aluminum stearate, alumina and aluminum laurate, iron oxide and iron stearate, zinc oxide and zinc stearate, silica and alumina and silicone, silica and alumina and aluminum stearate and silicone, triethanolamine, stearic acid or sodium hexametaphosphate and the like.

11. The cosmetic composition as claimed in claim 9, wherein the coated metal oxide nanopigments are silica-coated mixtures of titanium dioxide and cerium dioxide or alumina, silica and silicone-coated or alumina, silica and glycerin-coated mixtures of titanium dioxide and zinc dioxide.

12. The cosmetic composition as claimed in any one of claims 1 to 11, wherein the melanin pigments are obtained by oxidation of at least one indole compound, by oxidative or enzymatic polymerization of melanin precursors or by extraction of melanin from substances containing it.

13. The cosmetic composition as claimed in claim 12, wherein the melanin pigments are obtained by oxidation of at least one indole compound of formula 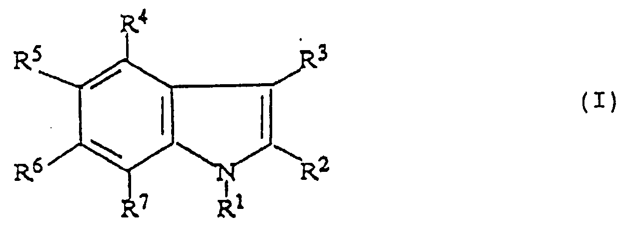 in which:
R¹ and R³ represent independently of each other a hydrogen atom or a C₁-C₄ alkyl group; R² represents a hydrogen atom, a C₁-C₄ alkyl group, a carboxyl group or a (C₁-C₄ alkoxy)carbonyl group; R⁴ and R⁷ represent independently of each other a hydrogen atom, a hydroxyl group, a C₁-C₄ alkyl group, an amino group, a C₁-C₄ alkoxy group, a (C₂-C₄ acyl)oxy group or a (C₂-C₄ acyl)amino group; R⁵ represents a hydrogen atom, a hydroxyl group, a C₁-C₄ alkoxy group, a C₁-C₄ alkyl group, a halogen atom, an amino group, a (C₂-C₁₄ acyl)oxy group, a (C₂-C₄ acyl)amino group or a trimethylsilyloxy group; R⁶ represents a hydrogen atom, a hydroxyl group, a C₁-C₄ alkoxy group, an amino group, a (C₂-C₄ acyl)oxy group, a (C₂-C₄ acyl)amino group, a trimethylsilyloxy group or a (C₂-C₄ hydroxyalkyl)amino group; it being possible for R⁵ and R⁶ also to form, together with the carbon atoms to which they are attached, a methylenedioxy ring which is optionally substituted by a C₁-C₄ alkyl or C₁-C₄ alkoxy group, or alternatively a carbonyldioxy ring; at least one of the radicals R⁴ to R⁷ represents an OZ or NHR⁰ group, at most one of the radicals R⁴ to R⁷ representing NHR⁰ and at most two of the radicals R⁴ to R⁷ representing OZ and, in the case where Z represents a hydrogen atom, the two OH groups are in positions 5 and 6; and at least one of the radicals R⁴ to R⁷ represents a hydrogen atom, and, in the case where only one of these radicals represents a hydrogen atom, only one radical among the radicals R⁴ to R⁷ then represents NHR⁰ or OZ, the other radicals representing a C₁-C₄ alkyl group; the radical R⁰ of the NHR⁰ group denoting a hydrogen atom, C₂-C₄ acyl or C₂-C₄ hydroxyalkyl group, and the radical Z of the OZ group denoting a hydrogen atom, a C₂-C₁₄ acyl group, a C₁-C₄ alkyl group or a trimethylsilyl group,
and their alkali metal, alkaline-earth metal, ammonium or amine salts, as well as the hydrochlorides, hydrobromides, sulfates and methanesulfonates of this indole compound.

14. The cosmetic composition as claimed in claim 13, wherein the indole compound is chosen from 4-hydroxyindole, 5-hydroxyindole, 6-hydroxyindole, 7-hydroxyindole, 4-hydroxy-5-methoxyindole, 4-hydroxy-5-ethoxyindole, 2-carboxy-5-hydroxyindole, 5-hydroxy-6-methoxyindole, 6-hydroxy-7-methoxyindole, 5-methoxy-6-hydroxyindole, 5,6-dihydroxyindole, N-methyl-5,6-dihydroxyindole, 2-methyl-5,6-dihydroxyindole, 3-methyl-5,6-dihydroxyindole, 2,3-dimethyl-5,6-dihydroxyindole, 2-carboxy-5,6-dihydroxyindole, 4-hydroxy-5-methylindole, 2-carboxy-6-hydroxyindole, 6-hydroxy-N-methylindole, 2-ethoxycarbonyl-5,6-dihydroxyindole, 4-hydroxy-7-methoxy-2,3-dimethylindole, 4-hydroxy-5-ethoxy-N-methylindole, 6-hydroxy-5-methoxy-2-methylindole, 6-hydroxy-5-methoxy-2,3-dimethylindole, 6-hydroxy-2-ethoxycarbonylindole, 7-hydroxy-3-methylindole, 5-hydroxy-6-methoxy-2,3-dimethylindole, 5-hydroxy-3-methylindole, 5-acetoxy-6-hydroxyindole, 5-hydroxy-2-ethoxycarbonylindole, 6-hydroxy-2-carboxy-5-methylindole, 6-hydroxy-2-ethoxycarbonyl-5-methoxyindole, 6-N-β-hydroxyethylaminoindole, 4-aminoindole, 5-aminoindole, 6-aminoindole, 7-aminoindole, N-methyl-6-β-hydroxyethylaminoindole, 6-amino-2,3-dimethylindole, 6-amino-2,3,4,5-tetramethylindole, 6-amino-2,3,4-trimethylindole, 6-amino-2,3,5-trimethylindole, 6-amino-2,3,6-trimethylindole, 5,6-diacetoxyindole, 5-methoxy-6-acetoxyindole, 5,6-dimethoxyindole, 5,6-methylenedioxyindole, 5,6-trimethylsilyloxyindole, the phosphoric ester of 5,6-dihydroxyindole, or from 5,6-dibenzyloxyindole, and from the addition salts of these compounds.

15. The cosmetic composition as claimed in claim 14, wherein the indole compound is 5,6-dihydroxyindole.

16. The cosmetic composition as claimed in any one of claims 1 to 15, wherein the melanin pigment is a synthetic composite melanin pigment deposited at the surface or incorporated into an inorganic or organic inert particulate filler.

17. The cosmetic composition as claimed in claim 16, wherein the composite melanin pigment is derived from the oxidation of at least one indole compound of formula (I) as claimed in claim 13, mixed with the particulate filler, in an essentially non-solvent medium of the said filler, at a temperature ranging from room temperature up to about 100°C, or alternatively is derived from the oxidative polymerization of melanin precursors on the particulate filler.

18. The cosmetic composition as claimed in claim 16 or 17, wherein the particulate filler consists of inert inorganic particles with a particle size of less than 20,000 nm, or of natural or synthetic, organic or inorganic polymers with a crystalline or amorphous crosslinked lattice which have a molecular weight of between 5,000 and 5,000,000.

19. The cosmetic composition as claimed in any one of claims 1 to 18, which is provided in the form of a lotion, thickened lotion, gel, cream, milk, powder, solid stick, foam or spray.

20. The cosmetic composition as claimed in claim 19, which contains, in addition, cosmetic adjuvants chosen from fatty substances, organic solvents, silicones, thickeners, demulcents, UV-A, UV-B or broad band sun-screen agents, antifoaming agents, moisturizing agents, perfumes, preservatives, surface-active agents, fillers, sequestrants, anionic, cationic, nonionic or amphoteric polymers or mixtures thereof, propellants, alkalizing or acidifying agents, colorants and pigments with a particle size greater than 100 nm.

21. The cosmetic composition as claimed in any one of claims 1 to 20, constituting a composition for protecting the human epidermis against ultraviolet rays, or anti-sun composition, which is provided in the form of a suspension or dispersion in solvents or fatty substances, in the form of an emulsion, in the form of an ointment, in the form of a gel, in the form of a solid stick or aerosol foam.

22. The cosmetic composition as claimed in any one of claims 1 to 20, used for protecting the hair against ultraviolet rays, which is provided in the form of a rinse-off shampoo, lotion , gel or composition to be applied before or after shampooing, before or after dyeing or bleaching, before, during or after permanent waving or hair straightening, in the form of a hair styling or treatment lotion or gel , a lotion or gel for blow drying or hair setting, hair lacquer , a composition for permanent waving or hair straightening , for dyeing or bleaching the hair.

23. The cosmetic composition as claimed in any one of claims 1 to 20, constituting a make-up for the eyelashes, the eyebrows, the skin or the hair, which is in the form of a cream for treating the epidermis, foundation, lipstick, eyeshadow, blusher, eyeliner, mascara or coloring gel and which is provided in anhydrous or aqueous solid or pasty form.

24. Non-therapeutic process for protecting the human epidermis and the hair against ultraviolet radiation which consists in applying to the skin or the hair an effective amount of a cosmetic composition as claimed in any one of claims 1 to 23.

25. Non-therapeutic use of melanin pigments derived from natural or synthetic sources, with a mean diameter of between 10 nm and 20,000 nm, for reducing or inhibiting the light-induced reaction of metal oxide nanopigments exposed to light, these metal oxides being chosen from titanium, zinc, cerium or zirconium oxides or mixtures thereof, with a mean diameter of less that 100 nm.

## Patentansprüche

1. Kosmetische Zusammensetzung,
dadurch **gekennzeichnet**, daß
sie, in Abmischung, mindestens ein Nanopigment von Metalloxiden aus den Oxiden von Titan, Zink, Cer, Zirkon oer aus deren Mischungen mit einem mittleren Durchmesser unterhalb 100 nm sowie mindestens ein Melaninpigment aus natürlichen oder synthetischen Quellen mit einem mittleren Durchmesser von 10 bis 20 000 nm in einem kosmetisch geeigneten Trägermedium enthält.

2. Kosmetische Zusammensetzung gemäß Anspruch 1,
dadurch **gekennzeichnet**, daß
die Nanopigmente der Metalloxide einen mittleren Durchmesser von 5 bis 50 nm aufweisen.

3. Kosmetische Zusammensetzung emäß Anspruch 1 oder 2,
dadurch **gekennzeichnet**, daß
das Metalloxid ein Titanoxid ist.

4. Kosmetische Zusammensetzung gemäß jedem Anspruch 1 oder 3,
dadurch **gekennzeichnet**, daß
die Melaninpigmente einen mittleren Durchmesser von 30 bis 15 000 nm aufweisen.

5. Kosmetische Zusammensetzung gemäß jedem der Ansprüche 1 bis 4,
dadurch **gekennzeichnet**, daß
die Nanopigmente der Metalloxide in der Zusammensetzung mit einer Konzentration von 0,1 bis 15 Gew.%, vorzugsweise von 0,5 bis 10 Gew.%, bezogen auf das Gesamtgewicht der Zusammensetzung, vorhanden sind.

6. Kosmetische Zusammensetzung gemäß jedem der Ansprüche 1 bis 5,
dadurch **gekennzeichnet**, daß
die Melaninpigmente in der Zusammensetzung mit einer Konzentration von 0,001 bis 2 Gew.%, vorzugswiese von 0,002 bis 1 Gew.%, bezogen auf das Gesamtgewicht der Zusammensetzung, vorhanden sind.

7. Kosmetische Zusammensetzung gemäß jedem der Ansprüche 1 bis 6,
dadurch **gekennzeichnet**, daß
das Gewichtsverhältnis Melaninpigmetn/Nanopigment von Metalloxid 0,00007 bis 10, vorzugsweise 0,001 bis 0,1, beträgt

8. Zusammensetzung gemäß jedem der Ansprüche 1 bis 7,
dadurch **gekennzeichnet**, daß
die Nanopigmente der Metalloxide nicht-umhüllte Pigmente sind.

9. Kosmetische Zusammensetzung gemäß jedem der Ansprüche 1 bis 7,
dadurch **gekennzeichnet**, daß
die Nanopigmente der Metalloxide umhüllte Pigmente sind, die einer oder mehreren Oberflächenbehandlungen chemischer, elektronischer, mechanochemischer oder mechanischer Natur mit Verbindungen unterzogen worden sind, ausgewählt aus Aminosäuren, Bienenwachs, Fettsäuren, Fettalkoholen, anionischen oberflächenaktiven Mitteln, Lecithinen, Fettsäuresalzen von Natrium, Kalium, Zink, Eisen oder Aluminium, Natriumhexametaphosphat, Metallalkoxiden, Polyethylen, Silikonen, Proteinen, Alkanolaminen, Siliziumoxiden und aus Metalloxiden.

10. Kosmetische Zusammensetzung gemäß Anspruch 9,
dadurch **gekennzeichnet**, daß
die Nanopigmente der umhüllten Metalloxide Pigmente von Titanoxiden sind, die umhüllt sind mit Siliziumdioxid, Siliziumdioxid und Aluminiumoxid, Siliziumdioxid und Eisenoxid, Aluminiumoxid und Silikon, Aluminiumoxid und Aluminiumstearat, Aluminiumoxid und Aluminiumlaurat, Eisenoxid und Eisenstearat, Zinkoxid und Zinkstearat, Siliziumdioxid und Aluminiumoxid und Silikon, Siliziumdioxid und Aluminiumoxid und Aluminiumstearat und Silikon, Triethanolamin, Stearinsäure oder Natriumhexametaphosphat.

11. Kosmetische Zusammensetzung gemäß Anspruch 9,
dadurch **gekennzeichnet**, daß
die Nanopigmente der umhülltem Metalloxide Mischungen aus Titandioxid und Ceroxid, die mit Siliziumdioxid umhüllt sind, oder Mischungen aus Titandioxid und Zinkdioxid sind, die mit Aluminiumoxid, Siliziumdioxid und Silikon oder mit Aluminiumoxid, Siliziumdioxid und Glycerin umhüllt sind.

12. Komsetische Zusammensetzung gemäß jedem der Ansprüche 1 bis 11,
dadurch **gekennzeichnet**, daß
die Melaninpigmente durch Oxidation mindestens einer Indolverbindung, durch oxidierende oder enzymatische Polymerisation von Melanin-Vorstufen oder durch Extraktion des Melanins aus dieses enthaltenden Substanzen erhältlich sind.

13. Kosmetische Zusammensetzung gemäß Anspruch 12,
dadurch **gekennzeichnet**, daß
die Melaninpigmente durch Oxidation mindestens einer Indolverbindung der Formel: 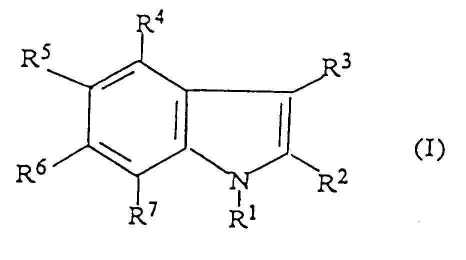 worin gilt:
- R¹ und R³ stellen, unabhängig voneinander, ein Wasserstoffatom oder eine C₁₋₄-Alkylgruppe dar;
- R² stellt ein Wasserstoffatom, eine C₁₋₄-Alkylgruppe, eine Carboxylgruppe oder eine C₁₋₄-Alkoxycarbonylgruppe dar;
- R⁴ und R⁷stellen, unabhängig voneinander, ein Wasserstoffatom, eine Hydroxy-, C₁₋₄-Alkyl-, Amino-, C₁₋₄-Alkoxy-, C₂₋₄-Acyloxy- oder eine C₂₋₄-Acylaminogruppe dar;
- R⁵ stellt ein Wasserstoffatom, eine Hydroxy-, C₁₋₄-Alkoxy-, C₁₋₄-Alkylgruppe, ein Halogenatom, eine Amino-, C₂₋₁₄-Acyloxy-, C₂₋₄-Acylamino- oder eine Trimethylsilyloxygruppe dar;
- R⁶ stellt ein Wasserstoffatom, eine Hydroxy-, C₁₋₄-Alkoxy-, Amino-, C₂₋₄-Acyloxy-, C₂₋₄-Acylamino-, Trimethylsilyloxy- oder eine C₂₋₄-Hydroxyalkylaminogruppe dar;
- R⁵ und R⁶ können auch zusammen mit den Kohlenstoffatomen, an die sie gebunden sind, einen Methylendioxyring, der gegebenenfalls mit einer C₁₋₄-Alkyl- oder C₁₋₄-Alkoxygruppe substituiert ist, oder auch einen Carbonyldioxyring bilden;
- mindestens einer der Reste R⁴ bis R⁷ stellt eine OZ- oder NHR⁰-Gruppe dar, wobei höchstens einer der Reste R⁴ bis R⁷ eine NHR⁰-Gruppe und höchstens zwei der Reste R⁴ bis R⁷ eine OZ-Gruppe darstellen, und in dem Fall, worin Z ein Wasserstoffatom dartellt, die beiden OH-Gruppen in den Positionen 5 und 6 vorliegen; und mindestens einer der Reste R⁴ bis R⁷ stellt ein Wasserstoffatom dar; und in dem Fall, worin ein einziger dieser Reste ein Wasserstoffatom darstellt, stellt ein einziger Rest aus den Resten R⁴ bis R⁷ dann eine NHR⁰- oder OZ-Gruppe dar, wobei die anderen Reste eine C₁₋₄-Alkylgruppe darstellen;
- der Rest R⁰ der NHR⁰-Gruppe bedeuten ein Wasserstoffatom, eine C₂₋₄-Acyl- oder C₂₋₄-Hydroxyalkylgruppe und der Rest Z der OZ-Gruppe ein Wasserstoffatom, eine C₂₋₁₄-Acyl-, C₁₋₄-Alkyl oder eine Trimethylsilylgruppe,
und durch Oxidation der Alkali-, Erdalkalimetall-, Ammonium- oder Aminsalze sowie der Hydrochloride, Hydrobromide, Sulfate und Methansulfonate dieser Indolverbindungen erhältlich sind.

14. Kosmetische Zusammensetzung gemäß Anspruch 13,
dadurch **gekennzeichnet**, daß
die Indolverbindung aus 4-Hydroxyindol, 5-Hydroxyindol, 6-Hydroxyindol, 7-Hydroxyindol, 4-Hydroxy-5-methoxyindol, 4-Hydroxy-5-ethoxyindol, 2-Carboxy-5-hydroxyindol, 5-Hydroxy-6-methoxyindol, 6-Hydroxy-7-methoxyindol, 5-Methoxy-6-hydroxyindol, 5,6-Dihydroxyindol, N-Methy-5,6-dihydroxyindol, 2-Methyl-5,6-dihydroxyindol, 3-Methyl-5,6-dihydroxyindol, 2,3-Dimethyl-5,6-dihydroxyindol, 2-Carboxy-5,6-dihydroxyindol, 4-Hydroxy-5-methylindol 2-Carboxy-6-hydroxyindol, 6-Hydroxy-N-methylindol, 2-Ethoxycarbonyl-5,6-dihydroxyindol, 4-Hydroxy-7-methoxy-2,3-dimethylindol, 4-Hydroxy-5-ethoxy-N-methylindol, 6-Hydroxy-5-methoxy-2-methylindol, 6-Hydroxy-5-methoxy-2,3-dimethylindol, 6-Hydroxy-2-ethoxycarbonylindol, 7-Hydroxy-3-methylindol, 5-Hydroxy-6-methoxy-2,3-dimethylindol, 5-Hydroxy-3-methylindol, 5-Acetoxy-6-hydroxyindol, 5-Hydroxy-2-ethoxycarbonylindol, 6-Hydroxy-2-carboxy-5-methylindol, 6-Hydroxyx-2-ethoxycarbonyl-5-methoxyindol, 6-N-β-Hydroxyethylaminoindol, 4-Aminoindol, 5-Aminoindol, 6-Aminoindol, 7-Aminoindol, N-Methyl-6-β-hydroxyethylaminoindol, 6-Amino-2,3-dimethylinol, 6-Amino-2,3,4,5-tetramethylindol, 6-Amino-2,3,4-trimethylindol, 6-Amino-2,3,5-trimethylindol, 6-Amino-2,3,6-trimthylindol, 5,6-Diacetoxyindol, 5-Methoxy-6-acetoxyindol, 5,6-Dimethoxyindol, 5,6-Methylendioxyindol, 5,6-Trimethylsilyloxyindol, Phosphorester von 5,6-Dihydroxyindol, 5,6-Dibenzyloxyindol und aus den Additionssalzen dieser Verbindungen ausgewählt ist.

15. Kosmetische Zusammensetzung gemäß Anspruch 14,
dadurch **gekennzeichnet**, daß
die Indolverbindung 5,6-Dihydroxyindol ist.

16. Kosmetische Zusammensetzung gemäß jedem der Ansprüche 1 bis 15,
dadurch **gekennzeichnet**, daß
das Melaninpigment ein synthetisches Verbund-Melaninpigment ist, wobei das Melanin auf der Oberfläche eines mineralischen oder organischen teilchenförmigen inerten Beladungsmittels abgeschieden oder darauf aufgebracht ist.

17. Kosmetische Zusammensetzung gemäß Anspruch 16,
dadurch **gekennzeichnet**, daß
das Verbund-Melaninpigment aus der Oxidation mindestens einer Indolverbindung der Formel (I) gemäß Anspruch 13, in Abmischung mit dem teilchenförmigen Beladungsmittel, in einem Medium, das im wesentlichen ein Nicht-Lösungsmittel für das genannte Beladungsmittel darstellt, bei einer Temperatur von Umgebungstemperatur bis ca. 100°C oder auch aus der oxidierenden Polymerisation von Melanin-Vorstufen auf dem teilchenförmigen Beladungsmittel stammt.

18. Kosmetische Zusammensetzung gemäß Anspruch 16 oder 17,
dadurch **gekennzeichnet**, daß
das teilchenförmige Beladungsmittel aus mineralischen inerten Teilchen einer Korngröße unterhalb 20 000 nm oder aus natürlichen oder snythetischen, organischen oder anorganischen Polymeren mit kristallinem oder amorphem Netzwerk mit einem Molekulargewicht von 5000 bis 5 000 000 zusammengesetzt ist.

19. Kosmetische Zusammensetzung gemäß jedem der Ansprüche 1 bis 18,
dadurch **gekennzeichnet**, daß
sie in Form einer Lotion, verdickten Lotion, eines Gels, einer Creme, Milch, eines Pulvers, Feststoffstäbchens, Schaums oder Spray vorliegt.

20. Kosmetische Zusammensetzung gemäß Anspruch 19,
dadurch **gekennzeichnet**, daß
sie ausserdem kosmetische Hilfstoffe enthält, ausgewählt aus Fettkörpern, organischen Lösunsmitteln, Silikonen, Verdickungsmitteln, Weichmachern, Sonnenfilterstoffen für UV-A, UV-B oder für einen großen Wellenlängenbereich, aus Antischaummitteln, hydratisierenden Mitteln, Parfüm-Produkten, Konservierungsstoffen, oberflächenaktiven Mitteln, Beladungsmitteln, Sequestriermitteln, anionischen, kationischen, nicht-ionischen, amphoteren Polymeren oder deren Mischungen, Treibmitteln, alkalisch oder sauer machenden Mitteln, Färbemitteln und aus Pigmenten einer Korngröße oberhalb 100 nm.

21. Kosmetische Zusammensetzung gemäß jedem der Ansprüche 1 bis 20,
die eine Zusammensetzung zum Schutz der menschlichen Haut vor ultravioletten Strahlen oder ein Sonnenschutzmittel darstellt,
dadurch **gekennzeichnet**, daß
sie in Form einer Suspension oder Dispersion in Lösungsmitteln oder Fettkörpern, in Form einer Emulsion, in Form einer Pommade, in Form eines Gels, in Form eines Feststofstäbchens oder als Aerosol-Schaum vorliegt.

22. Kosmetische Zusammensetzung gemäß jedem Anspruch 1 bis 20 zur Verwendung zum Schutz der Haare vor ultravioletten Strahlen,
dadurch **gekennzeichnet**, daß
sie in Form eines Shampoo, einer Lotion, eines Gels oder einer Zusammensetzung zum Spülen, zur Aufbingung vor oder nach dem Schamponieren, vor oder nach der Färbung oder Entfärbung, vor, während oder nach einer Dauerwelle oder einem Entfrisiervorgang, in Form einer Lotion oder eines Gels zum Frisieren oder zur Behandlung, einer Lotion oder eines Gels zum Bürsten oder zur Wellengebung, eines Lacks für die Haare, einer Zusammensetzung für die Dauerwelle oder zum Entfrisieren, zur Färbung oder Entfärbung der Haare vorliegt.

23. Kosmetische Zusammensetzung gemäß jedem der Ansprüche 1 bis 20,
die ein Produkt zum Schminken der Wimpern, Augenbrauen, der Haut oder der Haare darstellt,
dadurch **gekennzeichnet**, daß
sie in Form einer Creme zum Behandeln der Haut, einer Teint-Grundlage, eines Lippenstifts, einer Schminke für die Augenlider, einer Schminke für die Wangen, eines Liniermittels, auch "eye-liner" genannt, einer Wimperntusche oder eines Färbegels und in fester oder pasteuser, wasserfreier oder wässriger Form vorliegt.

24. Verfahren zum nicht-therapeutischen Schutz der menschlichen Haut und der Haare vor ultravioletter Strahlung,
dadurch **gekennzeichnet**, daß
man auf die Haut oder die Haare eine wirksame Menge einer kosmetischen Zusammensetzung gemäß jedem der Ansprüche 1 bis 23 aufträgt.

25. Nicht-therapeutische Verwendung von Melaninpigmenten aus natürlichen oder synthetischen Quellen mit einem Durchemsser von 10 bis 20 000 nm zur Verminderung oder Inhibierung einer fotoinduzierten Reaktion der Nanopigmente der Metalloxide, die der Einwirkung von Licht ausgesetzt sind, wobei die Metalloxide aus den Oxiden des Titans, Zink, Cers, Zirkons oder deren Mischungen mit einem mittleren Durchmesser unterhalb 100 nm ausgewählt sind.
